# EUROPEAN PATENT SPECIFICATION

(11) **EP 2 900 128 B1**
(45) Date of publication and mention of the grant of the patent: **13.07.2016**
(21) Application number: 13770645.3
(22) Date of filing: 18.09.2013
(51) Int. Cl.: A61B 5/024, A61B 5/0245, A61B 5/00, A61B 5/0416

(54) **PULSE METER FOR NEW-BORNS**
PULSMESSER FÜR NEUGEBORENE
COMPTEUR D'IMPULSIONS POUR NOUVEAUX-NÉS

(30) Priority: 26.09.2012 NO 20121097
(43) Date of publication of application: 05.08.2015
(73) Proprietor: Laerdal Medical AS, 4002 Stavanger (NO)
(72) Inventor: EILEVSTJØNN, Joar, N-4323 Sandnes (NO); MYKLEBUST, Helge, N-4025 Stavanger (NO); GOMO, Øystein, N-4085 Hundvåg (NO); MOLDEN, Mathias, N-Stavanger 4015 (NO); FOSSAN, Helge, N-4015 Stavanger (NO)
(74) Representative: Protector IP Consultants AS
(86) International application number: PCT/EP2013/069360
(87) International publication number: WO 2014/048807

(56) References cited:
- WO-A1-2009/074928
- WO-A1-2011/131234
- GB-A- 2 020 981
- JP-A- 2003 325 466
- US-A1- 2007 038 048
- US-A1- 2012 108 939

## Description

### Pulse meter for new-borns

The present invention relates to a pulse meter for measuring the pulse rate of a patient. Particularly, the present invention relates to a pulse meter for new born babies. The pulse meter has a simple structure and functioning mode and can be quickly and easily applied onto the torso/belly portion of a patient.

More particularly, the present invention relates to a pulse meter according to the preamble of claim 1.

### Background

Pulse meters for measuring the pulse of patients are widely known. For instance, pulse meters worn by athletes during exercise have become popular.

Pulse meters adapted for use by medical personnel need to be suitably constructed to be properly attachable to an appropriate portion of the patient's body. Hence, it is expected that pulse meters should have a simple configuration and should essentially be wearable in respect of a particular portion of the patient's body.

Patent publication US6928317 discloses a wearable heart rate transmitter, comprising a strap and a body. The strap is belt shaped. A conducting rubber plate extends on both ends of a central hole. The body part is installed inside the hole of the strap and the two contacting tips of the rubber plates are pressed at the two contacting points to contact the contacting tips inside the hole. Thus, the rubber plates contact the human body part to measure the heart beat.

This prior art pulse meter, as also other prior art pulse meters known in the art, exhibits complicated construction and working style.

Other known devices are JP 2003325466, US 2007/0038048, GB 2020981 and US 2007/123756.

Pulse meters known in the art are not able to address the requirement of quick and easy installation onto the torso portion of a human body.

The present invention provides a pulse meter which has at least two electrodes located on two arms which extend from a central portion, such that it can be fitted quickly and easily to the torso portion of a patient body as soon as the pulse rate is required to be measured.

It is the prime object of the present invention to provide a pulse meter which has a simple construction and functioning.

### The invention

According to the present invention, as defined in claim 1, there is provided a pulse meter comprising a first electrode and a second electrode adapted to be arranged in contact with a patient body part. The first and the second electrodes are arranged on a first arm and a second arm, respectively. The first and second arms extend out from a central portion, and respective first ends of the first and second arm are arranged to be bent away from each other when the pulse meter is being arranged into the applied position on the patient body part.

In an applied position on the patient body part the first and second arms are biased against each other with a force, wherein said force is transmitted from the first and second electrode to the patient body part.

According to a preferred embodiment, a third electrode is arranged in the position of the central portion and is adapted to contact the patient body part when the pulse meter is in the applied position. The third electrode will typically be arranged in a position on the body part between the first and second electrode. The first and second arm and the central portion can preferably together form a curved bow. In such an embodiment the first and second electrode are arranged to the curved bow and are facing inwards.

An actuator is advantageously connected to the inner side of the curved bow at two attachment sections, and a gap is present between the actuator and the curved bow. When compressing the actuator into the gap, towards the curved bow, the first and second arms will move away from each other, thereby making it possible to fit the pulse meter over a body part of the patient, typically over the torso of a newborn baby. Preferably the third electrode is arranged on the actuator.

In an alternative embodiment, the first arm and the second arm are connected with a hinge at the central portion.

Moreover, the first arm and the second arm are preferably continuous along said central portion. In such an embodiment the first and second arms can be parts of the same component (one single piece). Such a component can advantageously be a bow-shaped flexible piece, having a U-shape or a similar shape.

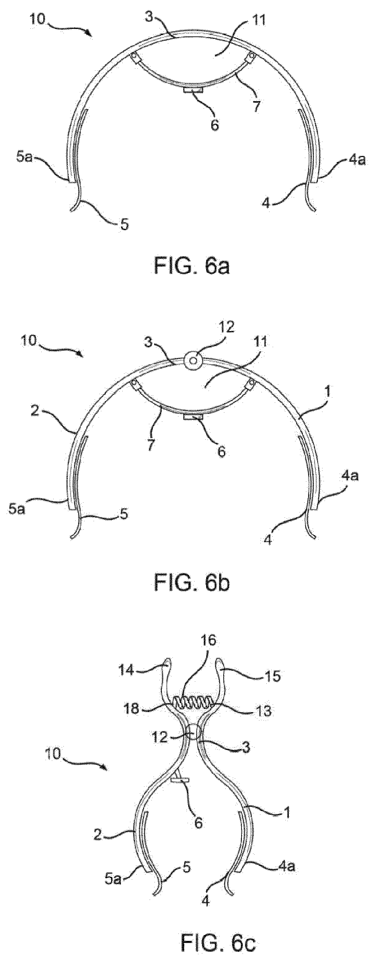

In yet an alternative embodiment of the pulse meter, the first arm and the second arm exhibit respective additional ends which are opposite of the first ends. In this embodiment the first and second arms are connected to each other with a hinge arranged between the first ends and the second ends, wherein a spring is arranged between the first arm and second arm in a position between the hinge and the additional ends. Such an embodiment is depicted in Fig. 6c.

Preferably, the first arm and the second arm are symmetrically disposed about the central portion.

The electrodes are preferably electrically connected to a wireless communication output device. In this manner the user, such as a midwife or other medical personnel, can read the measured pulse on a connected screen or numeric output display.

In a preferred embodiment a numeric output display is located on an external face of the pulse meter. In such an embodiment no additional equipment is needed in order to use the pulse meter. I.e. the measured pulse frequency is displaced on the pulse meter itself.

### Detailed example of embodiment

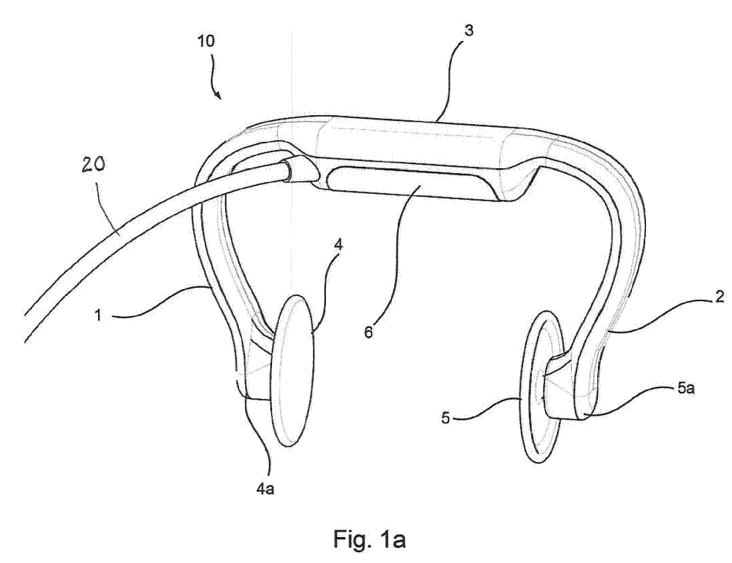

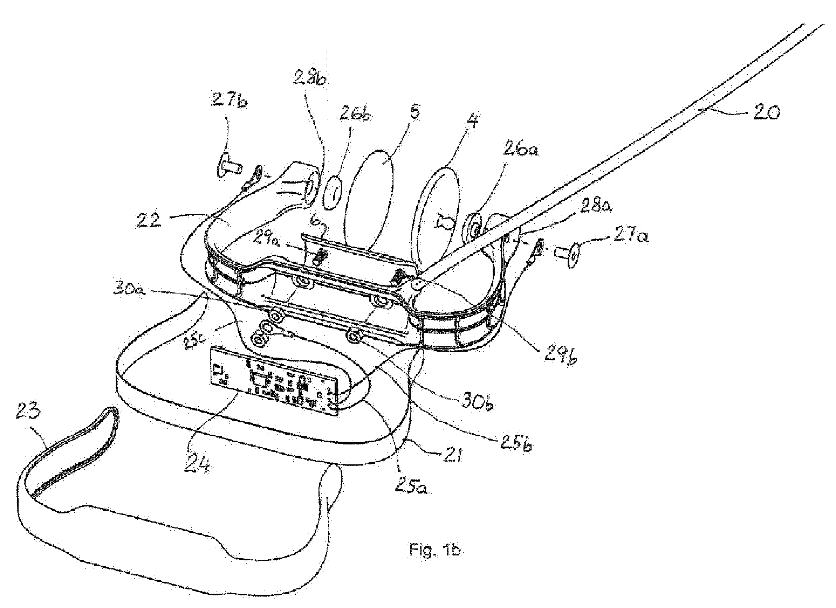

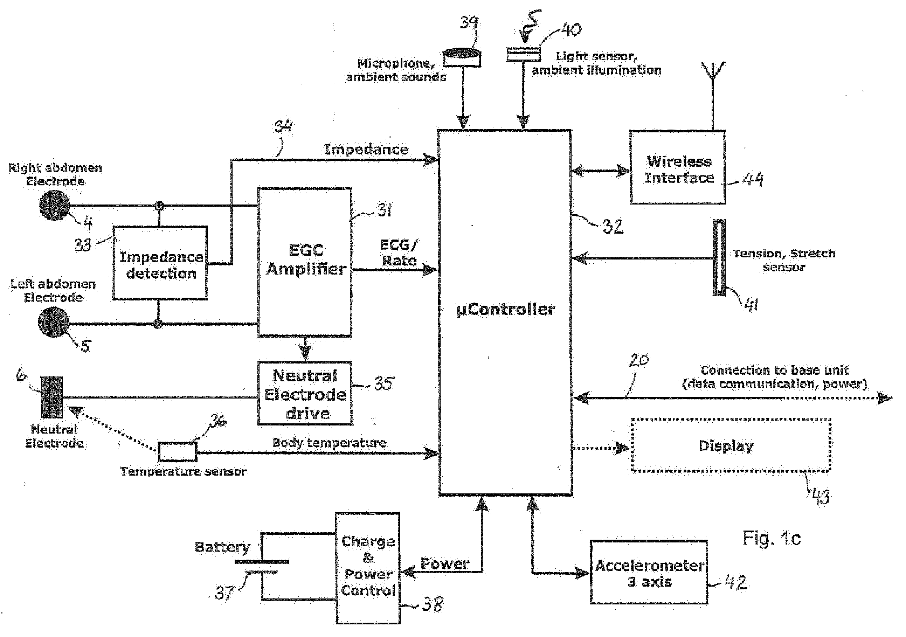

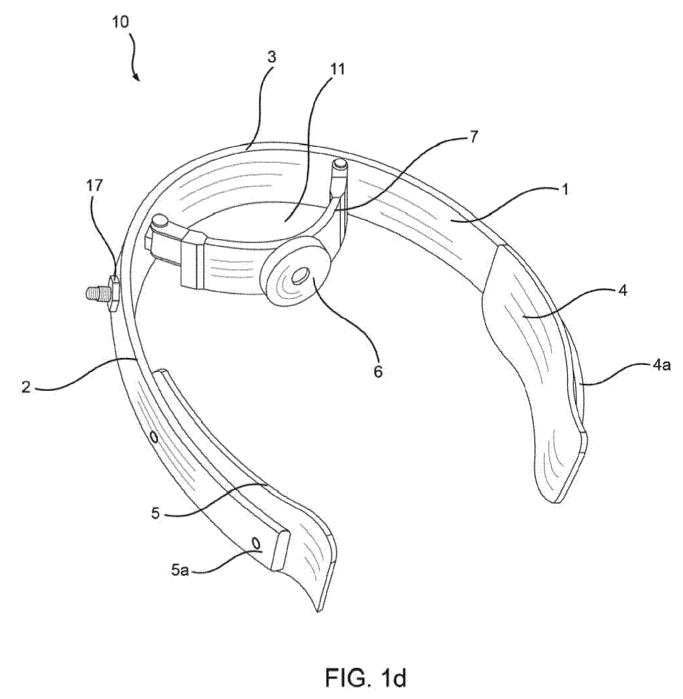

Having described the main features of the invention above, a more detailed and non-limiting description of some exemplary embodiments will be given in the following with reference to the drawings, in which
- Fig. 1 a: is a perspective view of the pulse meter according to a preferred embodiment;
- Fig. 1 b: is an exploded view of the pulse meter of figure 1 a;
- Fig. 1 c: is a block diagram illustrating the functions of the pulse meter;
- Fig 1d: is a perspective view of the pulse meter according to an alternative embodiment;
- Fig. 2: is a view of the preferred embodiment when opened for application onto a body part;
- Fig. 3: is a view of the preferred embodiment showing its application on a body part;
- Fig. 4: is a view of the preferred embodiment showing the withdrawal of the pulse meter;
- Fig. 5: is a view of the preferred embodiment showing the pulse meter in use on a body part;
- Fig. 6a: is a front view of the preferred embodiment of the pulse meter shown in Fig. 1;
- Fig. 6b: is a front view of another embodiment of the pulse meter; and
- Fig. 6c: is a front view of a further embodiment of the pulse meter of the present invention.

In the following some embodiments of the present invention are described. The embodiments are purely exemplary for the sake of understanding the invention and non-limiting.

When in the following it is referred to "above" or "below" and "top" and "bottom" "side", "inner", "outer" and similar terms, this is strictly referring to the embodiments as shown in the drawings.

It should also be understood that the orientation of the pulse meter may vary during application from what is shown in the drawings, without deviating from the principle of the invention.

In the figures like reference numerals represent corresponding features.

Fig. 1 a shows an isometric view of a preferred embodiment of the pulse meter 10 when not in use. It has a generally U-shaped bow and comprises a first arm 1 and a second arm 2, extending from a central portion 3. The arms 1, 2 and the central portion 3 are all made of a material that is easily flexible but quite resilient, such as nylon or plastic. The material may also be a flexible metal. Alternatively the arms may be stiff, but connected to the central portion by flexible sections. The first arm has a free end 4a and the second arm has a free end 5a. A first electrode 4 is arranged on the first arm 1 and a second electrode 5 is arranged on the second arm 2. A third electrode 6 is situated at the central portion 3, facing into the U-shape.

The first and second electrodes 1, 2 are preferably made of steel or another metal with good galvanic coupling. The faces of the electrodes that are to contact the body of the new-born have a relatively large flat area. They are preferably circular with a diameter of about 4 cm. The first and second electrodes 1, 2 are coupled to the U-shaped bow by snap fittings, e.g. of the push-button type. Thereby the electrodes are easy to remove for cleaning or replacement. It is also possible to replace the first and second electrodes 1, 2 with smaller, i.e. normal sized, electrodes if required. The surface of the flat area of the electrodes 1, 2 is smooth to allow for easy cleaning. When in use the electrodes are dry.

The third electrode is not crucial to the functioning of the pulse meter, but is advantageous as it is will provide a zero point for an applied common mode voltage and hence act to reduce noise.

The resilience of the U-shaped bow is configured so that the first and second electrodes press against the body of the new-born with about the same force as a stethoscope. The same procedure for cleaning as is for a stethoscope can also be used for cleaning the pulse meter of the present invention.

Referring to figure 1b, which shows the pulse meter in exploded view, the inner parts of the pulse meter will now be explained. The bow preferably has an inner core 21 made of spring steel. The spring steel core 21 preferably extends along the full length of the bow, and it is enclosed by plastic or rubber housing parts 22 and 23. Alternatively the bow may be has been covered with the resilient plastic or rubber in a moulding procedure. The spring steel is selected among the high quality steels, which does not deteriorate substantially with regard to spring resilience over time.

One of the housing parts 22 has a cavity within which a circuit board 24 is accommodated. The circuit board 24 has wires 25a, b and c, connecting the circuit board 24 with the three electrodes 4, 5 and 6. As explained above the electrodes 4 and 5 are attached to the bow by snap fit. To facilitated this a snap-button 26a, b for each electrode 4, 5 is attached by a rivet 27a, b, extending through a respective hole 28a, at the end of the bow. The rivet 27a, b may also be used to attach the wires 25b and c to the snap-buttons 26a, b.

The third electrode 6 may be attached by bolts 28a, b and nuts 30a, b. The wire 25a may be attached to the electrode 6 via one of the bolts 29a.

Figure 1c shows a block diagram of the pulse meter functions. The main electrodes 4 and 5 are connected to an EGC amplifier 31, which amplifies the signals from the electrodes and feeds it into a microcontroller 32. An impedance detector unit 33 is also coupled to the electrodes 4, 5 to detect impedance above a certain level (indicating that the electrodes does not have skin contact) and impedance below a certain level (indicating that the electrodes are short cut). A signal 34 is sent to the microcontroller 32 to set the microcontroller 32 in an appropriate mode. If the impedance level is outside a predetermined range, the microcontroller 32 will reject the heart rate measurements. An alarm may be initiated to alert the user. If the impedance is very high, this is a likely indication that the pulse meter is not placed on the baby. Consequently, the microcontroller may turn off the power to the meter after a certain delay.

The third (middle) electrode 6 is coupled to the EGC amplifier 31 via a neutral electrode drive unit 35. As mentioned above, the third electrode 6 is used to cancel out noise.

A temperature sensor 36 may also be integrated with the third electrode 6, or placed in close proximity with the electrode 6, in order to measure the skin temperature of the baby.

A battery 37 with a charge and power control unit 38 delivers the power to the microcontroller and associated circuitry.

A microphone 39 and light sensor 40 for detecting ambient sound and light is also coupled to the microcontroller 32. One or more tension sensors 41 are also coupled to the microcontroller 32. A 3-axis accelerometer 42 is coupled to the microcontroller 32. A display unit 43 may be coupled to the microcontroller 32, as well as a wireless transmitter 44. Alternatively, or in addition a wired connection 20 may be provided.

The function and applicability of the above various units will be explained more in detail below.

The pulse meter is preferably connected to a base unit (not shown) via a cable 20. Alternatively, it may be connected to the base unit wirelessly. However, for hospital use a wireless connection is less preferable, because the risk of interference with other types of instruments. If a wireless solution is chosen, the pulse meter will have an internal power source, such as a battery. The battery is preferably rechargeable, e.g. by connecting the meter to the base unit.

Preferably, the pulse meter and the base unit have an automatic on/off switch. The pulse meter may be adapted for attachment to the base unit when not in use. When the base unit senses that the pulse meter is attached to it, it will turn off, as will the pulse meter itself. As soon as the pulse meter is detached from the base unit, the system will turn itself on. This feature can be realised both in a wired and a wireless system. A convenient way of detecting if the pulse meter is attached to the base unit is by measuring the impedance between the first and second electrodes. The impedance measurement can also be used to detect if the pulse meter is attached to the baby. If substantially infinite impedance is detected over a set period of time, the system will assume that the pulse meter is not in contact with the body and it will turn itself off.

The base unit is equipped with a screen to display the readings of the pulse meter. A base unit with a screen is preferred over a screen on the pulse meter, since this enables pulse readings even when the new-born (and hence the pulse meter) is covered in blankets.

The pulse meter bow may also be equipped with means for measuring the amount of bending of the bow, e.g., by tension strips placed at the transition of the arms and the central portion or by measuring the distance between two arms arranged to open the bow. The distance can for instance be measures with a cord potentiometer, i.e. by attaching a potentiometer with a cord reel at one arm of the bow, preferably close to the central portion of the bow, and the outer end of the cord to the opposite arm. When the arms are bent outwards, the cord will turn the reel and hence alter the resistance of the potentiometer. A spring may be used to reel in the cord when the arms move towards one another again. A further alternative may be to use a light or laser source and a reflector to measure the distance as a function of the speed of light. It must then be ensured that nothing will block the light path during use.

By any of the above methods or other methods that are readily available by the person of skill, it is possible to measure chest diameter and chest expansion. Chest diameter can indicate the weight of the newborn, and may be used to provide a target for desired ventilation volume since target ventilation volumes are mostly indicated in millilitres per kg body weight. When the pulse meter is put across the torso of the new-born, the tension in the strips is automatically calibrated so that the minimum value of tension is designated as the value of empty lungs and the maximum tension is designated to the value of fully inflated lungs. Thereby the system may provide an estimate for chest expansion in approximate millilitres per ventilation. Although the ventilation volume may not be very accurate, at least the system will be able to count the number of ventilations per minute. If the chest expansion is reduced substantially compared to normal for a new-born, i.e. the tension variation frequency drops below a certain value, an alarm may be initiated to indicate that the baby has stopped breathing or is breathing too little. This may be as an audible sound or as a visual signal, or both. An alarm may also be initiated if the breathing frequency increases substantially, as this may be an indication of hyperventilation.

An alternative way of detecting that the pulse meter is not attached to the body, is by monitoring the tension strips. If the strips are in idle mode, i.e. not stretched, for a prolonged time, the pulse meter may be considered not to be in contact with the body of the patient.

As an alternative or additional feature, the pulse meter may also be equipped with one or more accelerometers. The accelerometers may be used independently to detect chest expansion or may be used in co-operation with the tension strips to calibrate these. The accelerometers are also suitable for detecting other types of movement of the baby, such as spasms, handling of the baby and stimulation movements during resuscitation.

Chest expansion may also be detected by impedance measurements. The impedance imposed to detect this will be of a much higher frequency that the ECG measurements in order not to interfere with these.

The pulse meter may also have one or more temperature sensors to detect temperature changes of the baby. The temperature sensor will probably not be able to measure the absolute temperature, but will be able to detect if the baby has become colder or hotter than initially.

The pulse meter can also have at least one photodiode or similar light sensor to detect if the new-born is covered in a blanket or not. New-borns, especially premature, will easily get cold if they are not covered by a blanket or similar. This feature will provide an extra safety against a baby being left without a cover.

The pulse meter may also be provided with a microphone to detect sounds from the baby, e.g. cries or whimpers. This sound may be communicated to the base unit if this is close to medical personnel or to another receiver. A filter for cancelling out other sounds from the surroundings may also be present. Alternatively to transmitting the actual baby sound to the base unit, the base unit may also give an audible prompt indicating that the baby is crying.

With this system it is possible to measure and detect several functions as described above. It can detect if the baby is breathing or having apnoea. This is of particular importance in developing countries where new-borns may be lying for a prolonged time without continuous supervision. If a condition is detected that may be critical, or if a rapid change in condition is detected, an alarm may go off to attract the attention of the medical personnel nearby.

The pulse meter, or preferably the base unit, may also be equipped with a clock display. This is particularly useful in developing countries where medical personnel often do not have a watch. The clock may also have a stop watch function to facilitate manual pulse taking.

The base unit may also be adapted for connection to other types of medical equipment, such as an oxygen sensor for determining oxygen saturation. Thereby a more complete picture of the condition of the new-born can be determined and correlations between different measurements can be facilitated.

Fig. 1d shows an isometric view of an alternative embodiment of the pulse meter 10 when not in use. It comprises a first arm 1 and a second arm 2, extending from the central portion 3. The arms 1, 2 and the central portion 3 are all made of a material that is easily flexible but quite resilient, such as nylon or plastic. The material may also be a flexible metal. The first arm has a free end 4a and the second arm has a free end 5a. A first electrode 4 is arranged on the first arm 1 and a second electrode 5 is arranged on the second arm 2.

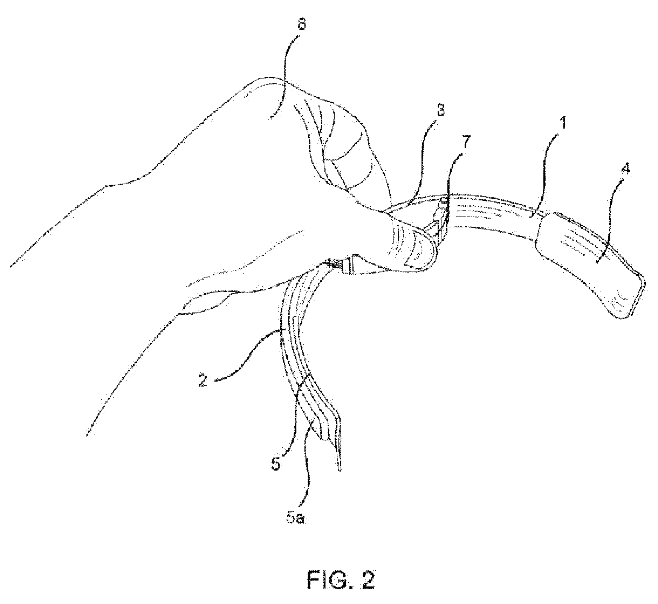

Fig. 2 shows the pulse meter 10 in an opened position, ready to be placed over a body part of a patient, such as the torso of a new-born baby. There is an actuator 7 which is flexible. The two ends of the actuator 7 are attached to the first arm 1 and the second arm 2 at portions away from the free ends 4a, 5a and also away from the first and second electrode 4, 5. These attachment locations of the two ends of the actuator 7 are on the inner side of a curved shape of the pulse meter 10, near the central portion 3. The actuator 7 should be flexible so that it bends into a less curved shape when forced towards the central portion 3 of the pulse meter 10.

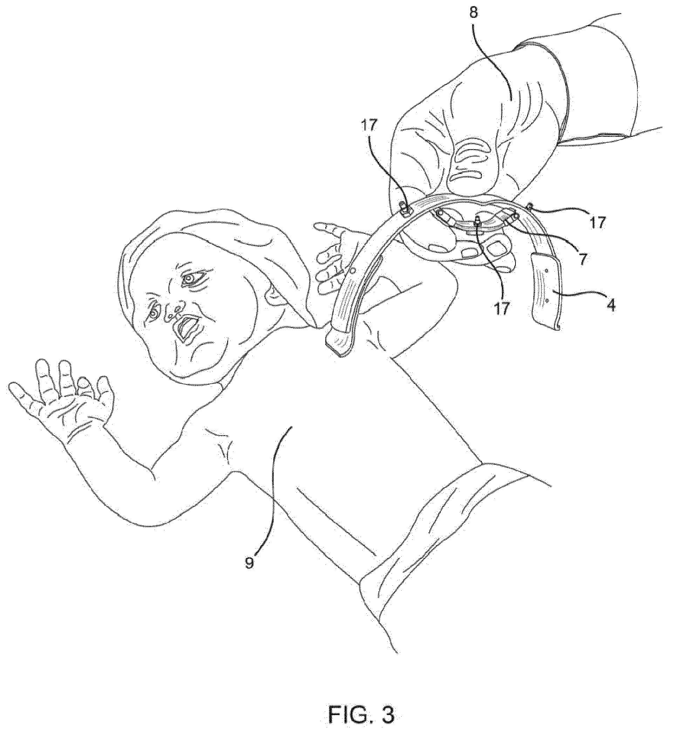

Fig. 3 shows the pulse meter 10 in the same position as in Fig. 2, however shown in relation to the body part 9 of a new-born baby onto which the pulse meter shall be applied.

Alternatively, one of the ends of the actuator 7 may be slidably attached to one of the arms 1, 2 of the pulse meter 10 and the other end may be fixedly attached. In such an embodiment the actuator 7 should be stiffer in order to provide an expanding mutual movement between the first and second arm 1, 2.

There is a gap 11 between the inner side of the central portion 3 and the actuator 7, cf. Fig. 1d.

It can also be seen from Fig. 1 that the pulse meter 10 is continuous along the central portion 3, forming a curved bow.

A third electrode 6 (Fig. 1) is located on the actuator 7, close to the central portion 3. All the electrodes 4, 5, 6 are located at the inner side of the curved shape / bow of the pulse meter 10. The third electrode 6 is not absolutely essential for the pulse meter to operate. It is used essentially for reducing noise from the measured signals. The electrodes are preferably made of steel.

The structure of the pulse meter 10 shown in Fig. 1 is such that it is flexible and that the central portion 3, the first arm 1 and the second arm 2 form a curved bow. The first electrode 4 and second electrode 5 are arranged on the curved bow and are facing inward. In the shown embodiment, the first electrode 4 and second electrode 5 are arranged in close proximity to the free ends 4a, 5a.

There is a circuitry (not shown) connected to the electrodes which is adapted to provide output signals to the user. In one embodiment the output signal may be transmitted by wireless communication to a user interface, such as a computer screen or a simple numeric display.

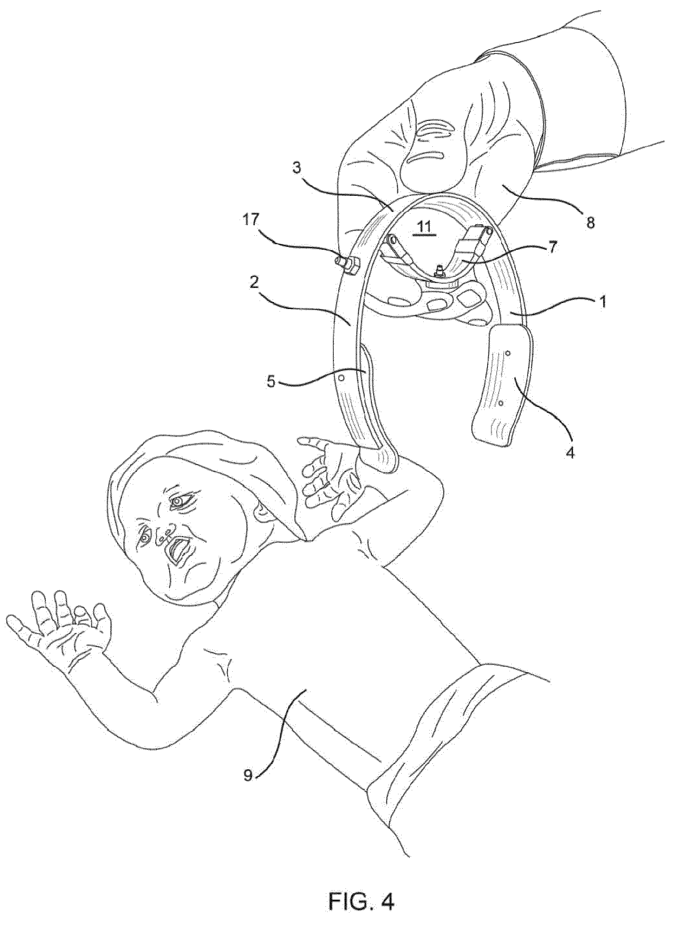

In an alternative embodiment each electrode can connect to an electric wire through electric connectors 17 (visible in Fig. 3 and Fig. 4). The circuitry may then be arranged in another position than on the pulse meter 10.

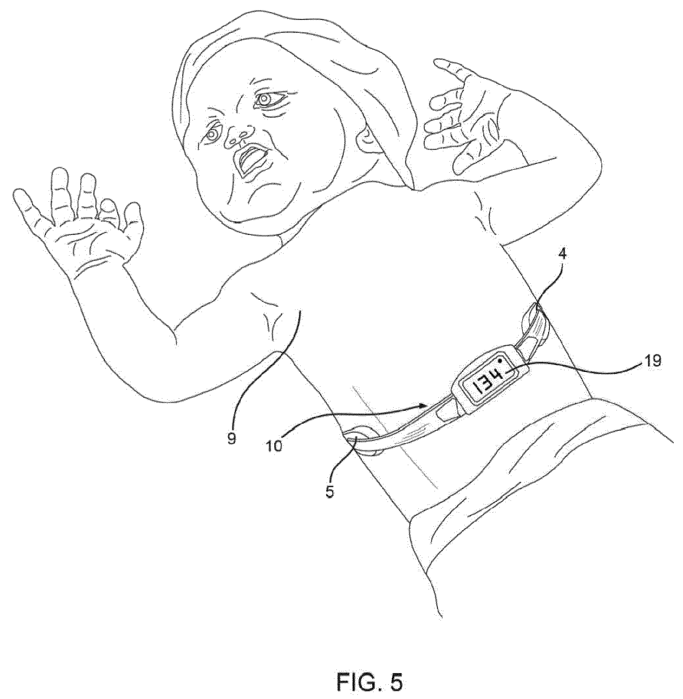

In a further alternative embodiment, the pulse meter 10 comprises both electric circuitry connected to three electrodes 4, 5, 6 as well as a numeric display 19 arranged for instance on the central portion 3, on the external side of the curved bow shape. Such an embodiment is shown in Fig. 5. In this drawing the actuator 7 is not visible, as it is arranged between the central portion 3 (having the numeric display 19), and the body part 9 of the patient. A pulse meter 10 according to such an embodiment needs to be provided with electrical power, such as with a battery.

Fig. 2 to Fig. 5 are views of the pulse meter 10 of Fig. 1, when used by a midwife or any other user. These figures will be referred to again while explaining the functioning of the preferred embodiment.

Fig. 6a is a schematic side view of the pulse meter 10 as shown in Fig. 1.

Fig. 6b is a side view of another embodiment of the pulse meter 10 where the first arm 1 and second arm 2 are connected by a hinge 12 at the central portion 3 of the pulse meter. The other features which are the same as in Fig. 1 and are not repeated for the sake of brevity. It is noted that in this embodiment, when incorporating a hinged connection 12 between the first and second arm 1, 2, the first and second arms 1, 2 do not have to exhibit inherent flexibility. The flexibility and the resilient characteristic can be provided by the hinge 12 and flexibility of the actuator 7. It may also be provided by a biased hinge 12, such as with a spring (not shown).

Fig. 6c is another embodiment of the pulse meter 10. The first arm 1 and the second arm 2 are connected to each other at the hinge 12 at the central portion 3. The first arm 1 and the second arm 2 have two additional free ends 14, 15 on their top portions, above the hinge 12, for gripping and operating the pulse meter. A spring 16 is located between the first arm 1 and the second arm 2 above the hinge 12 and is attached to the first arm 1 and second arm 2, at points 13, 18 near the additional free ends 14, 15. The third electrode 6 is located on the second arm 2 at a suitable point below the hinge 12 so that proper contact with the patient's body is ensured, at a position between the first and second electrode 4, 5. It may in stead be located on the first arm 1, near the central portion 3.

In the embodiment shown in Fig. 6c, the arms 1, 2 may be of a material with substantially no spring action and the spring action is then provided by the spring 16. The additional ends 14, 15 are compressed by the user so that the spring 16 is also consequentially compressed and the additional ends 14, 15 approach each other. In that event, the free ends 4a, 5a move away from each other by means of the hinge joint 12.

In this open condition, the pulse meter 10 is placed on the patient's body 9 with the pressure still applied on the spring 16. Due to spring action, the two arms 1, 2 are always trying to regain their original position, so when the pressure on spring 16 is released, the arms 1, 2 along with the electrodes 4, 5 fixed on them, firmly press upon the patient's body by spring force. To be precise, once the compression is released, the additional free ends 14, 15 move away from each other due to spring effect and the free ends 4a and 5a move towards each other, facing inwardly.

In all the embodiments shown in the drawings, the first arm 1 and the second arm 2 are symmetrically disposed about the central portion 3 of the pulse meter 10. However, this is not mandatory for achieving the objects of the present invention.

The functioning of the pulse meter 10 shown in Fig. 1b to Fig. 5 will now be explained further.

The actuator 7 is compressed by the user 8 as shown in Fig. 2 for opening the pulse meter 10, so that the two free ends 4a, 5a along with the first electrode 4 and the second electrode 5 move away from each other. Hence, pressing the actuator 7 towards the central portion 3 opens up the pulse meter 10.

In this open condition, the pulse meter is placed on the patient's body part 9 with the pressure still applied on the actuator 7. Due to the resilience or spring action of the material, the two arms 1 and 2 are always trying to regain their original form. So, when the pressure on the actuator 7 is released, the arms 1, 2 along with the electrodes 4, 5 fixed on them, firmly press upon the patient's body part 9 by spring force.

Fig. 3 shows how easily the user 8 holds the pulse meter 10 in open position (as also shown in Fig. 2) and easily places it over the abdomen of the torso 9 of a new-born with one hand. This becomes easy because the two ends 4a, 5a have moved away from each other. Hence, the pulse meter 10 can be installed quickly with one single hand as soon as the pulse rate needs to be recorded.

It is noteworthy, that the gap 11 between the inner side of the central portion 3 and the actuator 7 is minimized when the user compresses the actuator 7. This is the utility of the gap 11. It makes the compression of the actuator 7 by the user 8 possible. When releasing the grip from the actuator 7, the third electrode 6 will be moved towards the body part 9 of the patient as the actuator 7 moves towards its original and more curved shape/position (as shown in Fig. 1). In applied mode, the three electrodes 4, 5, 6 are in sufficient contact with the body part 9.

Fig. 4 shows that the pulse meter has been withdrawn from the body part 9. The actuator 7 has been released causing the first and second arm 1, 2 and the electrodes 4, 5 to move towards each other.

The present invention has been described with reference to some embodiments for the sake of understanding only and it should be clear to persons skilled in the art that the present invention includes a number of modifications within the scope of what is claimed in the appended claims. Especially, the features explained with regard to figure 1 can also readily be implemented in the embodiments of figures 1b to 5.

## Claims

1. Pulse meter comprising a first electrode (4) and second electrode (5) adapted to be arranged in contact with a patient body part (9),
the first and the second electrode (4, 5) being arranged on a first arm (1) and a second arm (2), respectively, the first and second arm (1, 2) extending out from a central portion (3), and the respective first ends (4a, 5a) of the first and second arm (1, 2) are arranged to be bent away from each other when the pulse meter (10) is being arranged into the applied position on the patient body part (9), **characterized in that** the first and second arm (1, 2) and the central portion (3) together form a curved bow, the curved bow is equipped with means, such as at least one tension strip, cord potentiometer, light or laser emitter and reflector, for measuring the amount of bending of the bow or the distance between the arms of the bow, and thereby monitoring the breathing of the patient, that the first and second electrode (4, 5) are arranged to the curved bow and are facing inwards, that in an applied position on the patient body part (9), the first and second arms (1, 2) are biased against each other with a force, wherein said force is inflicted from the first and second electrode (4, 5) to the patient body part (9).

2. Pulse meter according to claim 1, **characterized in that** a third electrode (6) is arranged in the position of the central portion (3) and is adapted to contact the patient body part (9) when the pulse meter (10) is in the applied position.

3. Pulse meter according to claim 1 or 2, **characterized in that** the first and second electrodes (4, 5) are snap-fit to their respective arm (1, 3).

4. Pulse meter according to any of the preceding claims, **characterized in that** the first and second electrodes (4, 5) are of metal, preferably steel, or other material with good galvanic coupling.

5. Pulse meter according to any of the preceding claims, **characterized in that** the first and second electrodes (4, 5) have a generally flat surface for contact with the skin of the patient that is between 7 and 19 cm².

6. Pulse meter according to one of the preceding claims, **characterized in that** it comprises at least one accelerometer.

7. Pulse meter according to one of the preceding claims, **characterized in that** it comprises a means for imposing a frequency between the electrodes that is substantially higher than the frequency used for pulse measurement.

8. Pulse meter according to one of the preceding claims, **characterized in that** it comprises at least one temperature sensor.

9. Pulse meter according to one of the preceding claims, **characterized in that** it comprises at least one light sensor to detect if the patient is covered.

10. Pulse meter according to one of the preceding claims, **characterized in that** it comprises a microphone, which is adapted to communicate with a remote receiver.

11. Pulse meter according to one of the preceding claims, **characterized in that** it connected by wire or wirelessly to a base unit, which base unit comprises an interface means for providing feedback to a caretaker, such as a display, light or sound generating means.

12. Pulse meter according to claim 11, **characterized in that** it is adapted to be attached to the base unit when not in use, and that it comprises means for switching on or off the power in response to the pulse meter being attached to the base unit or not, e.g. by measuring impedance over the first and second electrodes.

13. Pulse meter according to one of the preceding claims, **characterized in that** it comprises means for detecting substantially infinite impedance between the first and second electrodes and for switching off the pulse meter if substantially infinite impedance is detected over a certain time.

14. Pulse meter according to one of the preceding claims, **characterized in that** it comprises means for switching off the pulse meter if the tension strip is in a non-streched mode for a prolonged time.

15. Pulse meter according to any of the claims 4 - 16, **characterized in that** an actuator (7) is connected to the inner side of the curved bow at two attachment sections, and wherein a gap (11) is present between the actuator (7) and the curved bow.

16. Pulse meter according to claim 15, **characterized in that** the third electrode (6) is arranged on the actuator (7).

17. Pulse meter according to any of the preceding claims, **characterized in that** said first arm (1) and said second arm (2) are connected with a hinge (12) at said central portion (3).

18. Pulse meter according to any of claims 1 to 16, **characterized in that** said first arm (1) and said second arm (2) are continuous along said central portion (3).

19. Pulse meter according to any one of claims 1 to 17, **characterized in that** the first arm (1) and the second arm (2) exhibit respective second ends (14,15) which are opposite of the first ends (4a, 5a) and that the first and second arm (1, 2) are connected to each other with a hinge (12) arranged between the first ends (4a, 5a) and the second ends (14, 15), wherein a spring (16) is arranged between the first arm (1) and second arm (2) in a position between the hinge (12) and the second ends (14, 15).

20. The pulse meter according to any one of the preceding claims, **characterized in that** said first arm (1) and said second arm (2) are symmetrically disposed about said central portion (3).

21. The pulse meter according to any one of the preceding claims, **characterized in that** the electrodes (4, 5, 6) are electrically connected to a wireless communication output device.

22. The pulse meter according to any one of the preceding claims, **characterized in that** a numeric output display (19) is located on an external face of the pulse meter (10).

## Patentansprüche

1. Pulsmesser, umfassend eine erste Elektrode (4) und zweite Elektrode (5), welche dazu ausgelegt sind, in Kontakt mit einem Patientenkörperteil (9) angeordnet zu werden,
wobei die erste und die zweite Elektrode (4, 5) jeweils an einem ersten Arm (1) und einem zweiten Arm (2) angeordnet werden, wobei der erste und zweite Arm (1, 2) sich von einem zentralen Abschnitt (3) erstrecken, und die jeweiligen ersten Enden (4a, 5a) des ersten und zweiten Arms (1, 2) so angeordnet sind, um voneinander weg gebogen zu werden, wenn der Pulsmesser (10) in der angelegten Position auf dem Patientenkörperteil (9) angeordnet ist, **dadurch gekennzeichnet, dass** der erste und zweite Arm (1, 2) und der zentrale Abschnitt (3) zusammen einen gekrümmten Bogen bilden, wobei der gekrümmte Bogen mit Mitteln, wie zumindest einem Spannband, einem Kabelpotentiometer, Licht- oder Laseremitter und -reflektor, zum Messen des Biegebetrags des Bogens oder des Abstands zwischen den Armen ausgestattet ist, und dadurch die Atmung des Patienten überwacht wird, dass die erste und die zweite Elektrode (4, 5) an dem gekrümmten Bogen angeordnet sind und nach innen zeigen, dass in einer angelegten Position auf dem Patientenkörperteil (9) der erste und zweite Arm (1, 2) mit einer Kraft gegeneinander vorgespannt sind, wobei die Kraft von der ersten und zweiten Elektrode (4, 5) auf das Patientenkörperteil (9) ausgeübt wird.

2. Pulsmesser nach Anspruch 1, **dadurch gekennzeichnet, dass** eine dritte Elektrode (6) in der Position des zentralen Abschnitts (3) angeordnet und dazu ausgelegt ist, das Patientenkörperteil (9) zu kontaktieren, wenn der Pulsmesser (10) in der angelegten Position ist.

3. Pulsmesser nach Anspruch 1 oder 2, **dadurch gekennzeichnet, dass** die erste und zweite Elektrode (4, 5) auf ihre jeweiligen Arme (1, 3) eingerastet sind.

4. Pulsmesser nach einem der vorhergehenden Ansprüche, **dadurch gekennzeichnet, dass** die erste und zweite Elektrode (4, 5) aus Metall, vorzugsweise Stahl, oder einem anderen Material mit guter galvanischer Kopplung sind.

5. Pulsmesser nach einem der vorhergehenden Ansprüche, **dadurch gekennzeichnet, dass** die erste und zweite Elektrode (4, 5) eine hauptsächlich flache Oberfläche zum Kontaktieren der Haut des Patienten aufweist, welche zwischen 7 und 19 cm² ist.

6. Pulsmesser nach einem der vorhergehenden Ansprüche, **dadurch gekennzeichnet, dass** er zumindest einen Beschleunigungsmesser umfasst.

7. Pulsmesser nach einem der vorhergehenden Ansprüche, **dadurch gekennzeichnet, dass** er ein Mittel zum Anlegen einer Frequenz zwischen den Elektroden umfasst, die wesentlich größer als die zur Pulsmessung verwendete Frequenz ist.

8. Pulsmesser nach einem der vorhergehenden Ansprüche, **dadurch gekennzeichnet, dass** er zumindest einen Temperatursensor umfasst.

9. Pulsmesser nach einem der vorhergehenden Ansprüche, **dadurch gekennzeichnet, dass** er zumindest einen Lichtsensor umfasst, um zu erkennen, ob der Patient abgedeckt ist.

10. Pulsmesser nach einem der vorhergehenden Ansprüche, **dadurch gekennzeichnet, dass** er ein Mikrofon umfasst, welches dazu ausgelegt ist, mit einem Fernsteuerempfänger zu kommunizieren.

11. Pulsmesser nach einem der vorhergehenden Ansprüche, **dadurch gekennzeichnet, dass** er über einen Draht oder drahtlos mit einer Basiseinheit verbunden ist, wobei die Basiseinheit ein Schnittstellenmittel, wie ein Display, Licht oder Ton erzeugendes Mittel, umfasst, um eine Rückmeldung an einen Pfleger bereitzustellen.

12. Pulsmesser nach Anspruch 11, **dadurch gekennzeichnet, dass** er, wenn er nicht in Gebrauch ist, zum Befestigen an einer Basiseinheit ausgelegt ist, und dass er Mittel zum An- oder Ausschalten der Leistung als Antwort darauf umfasst, ob der Pulsmesser an der Basiseinheit befestigt ist oder nicht, z. B. durch Messen der Impedanz über die erste und zweite Elektrode.

13. Pulsmesser nach einem der vorhergehenden Ansprüche, **dadurch gekennzeichnet, dass** er Mittel zum Erfassen einer im Wesentlichen unendlichen Impedanz zwischen der ersten und zweiten Elektrode und zum Ausschalten des Pulsmessers umfasst, wenn eine im Wesentlichen unendliche Impedanz über eine bestimmte Zeit erfasst wird.

14. Pulsmesser nach einem der vorhergehenden Ansprüche, **dadurch gekennzeichnet, dass** er Mittel zum Ausschalten des Pulsmessers umfasst, wenn sich das Spannband über längere Zeit in einem nicht-gespannten Zustand befindet.

15. Pulsmesser nach einem der Ansprüche 4 - 16, **dadurch gekennzeichnet, dass** ein Aktuator (7) mit der Innenseite des gekrümmten Bogens an zwei Befestigungsabschnitten verbunden ist, und wobei ein Spalt (11) zwischen dem Aktuator (7) und dem gekrümmten Bogen vorhanden ist.

16. Pulsmesser nach Anspruch 16, **dadurch gekennzeichnet, dass** die dritte Elektrode (6) auf dem Aktuator (7) angeordnet ist.

17. Pulsmesser nach einem der vorhergehenden Ansprüche, **dadurch gekennzeichnet, dass** der erste Arm (1) und der zweite Arm (2) mit einem Scharnier (12) am zentralen Abschnitt (3) verbunden sind.

18. Pulsmesser nach einem der Ansprüche 1 bis 16, **dadurch gekennzeichnet, dass** der erste Arm (1) und der zweite Arm (2) entlang des zentralen Abschnitts (3) ununterbrochen sind.

19. Pulsmesser nach einem der Ansprüche 1 bis 17, **dadurch gekennzeichnet, dass** der erste Arm (1) und der zweite Arm (2) jeweils zusätzliche Enden (14, 15) aufweisen, welche den ersten Enden (4a, 5a) gegenüberliegen, und dass der erste und zweite Arm (1, 2) jeweils miteinander über ein Scharnier (12) verbunden sind, welches zwischen den ersten Enden (4a, 5a) und den zweiten Enden (14, 15) angeordnet ist, wobei eine Feder (16) zwischen dem ersten Arm (1) und dem zweiten Arm (2) in einer Position zwischen dem Scharnier (12) und den zusätzlichen Enden (14, 15) angeordnet ist.

20. Pulsmesser nach einem der vorhergehenden Ansprüche, **dadurch gekennzeichnet, dass** der erste Arm (1) und der zweite Arm (2) symmetrisch bezüglich des zentralen Abschnitts (3) angeordnet sind.

21. Pulsmesser nach einem der vorhergehenden Ansprüche, **dadurch gekennzeichnet, dass** die Elektroden (4, 5, 6) elektrisch mit einer drahtlosen Kommunikationsausgabeeinrichtung verbunden sind.

22. Pulsmesser nach einem der vorhergehenden Ansprüche, **dadurch gekennzeichnet, dass** eine nummerische Ausgabeanzeige an einer externen Seite des Pulsmessers (10) angeordnet ist.

## Revendications

1. Compteur d'impulsions comprenant une première électrode (4) et une deuxième électrode (5) adaptées pour être agencées en contact avec une partie de corps d'un patient (9),
les première et deuxième électrodes (4, 5) étant respectivement agencées sur un premier bras (1) et un deuxième bras (2), les premier et second bras (1, 2) s'étendant vers l'extérieur depuis une partie centrale (3), et les premières extrémités respectives (4a, 5a) des premier et deuxième bras (1, 2) sont agencées pour se plier en s'éloignant l'une de l'autre lorsque le compteur d'impulsions (10) est agencé dans la position appliquée sur la partie de corps du patient (9), **caractérisé en ce que** les premier et deuxième bras (1, 2) et la partie centrale (3) forment ensemble un arc incurvé, l'arc incurvé est équipé d'un moyen, tel qu'au moins une bande de tension, un potentiomètre à fil, un réflecteur et un émetteur de lumière ou laser, pour mesurer la quantité de pliage de l'arc ou la distance entre les bras de l'arc, et contrôler ainsi la respiration du patient, **en ce que** les première et deuxième électrodes (4, 5) sont agencées sur l'arc incurvé et sont tournées vers l'intérieur, **en ce que**, dans une position appliquée sur la partie de corps du patient (9), les premier et deuxième bras (1, 2) sont poussés l'un contre l'autre avec une force, dans lequel ladite force est infligée depuis les première et deuxième électrodes (4, 5) à la partie de corps du patient (9).

2. Compteur d'impulsions selon la revendication 1, **caractérisé en ce qu'**une troisième électrode (6) est agencée dans la position de la partie centrale (3) et est adaptée pour être en contact avec la partie de corps du patient (9) quand le compteur d'impulsions (10) est dans la position appliquée.

3. Compteur d'impulsions selon la revendication 1 ou 2, **caractérisé en ce que** les première et deuxième électrodes (4, 5) sont encliquetées sur leur bras respectif (1, 3).

4. Compteur d'impulsions selon l'une quelconque des revendications précédentes, **caractérisé en ce que** les première et deuxième électrodes (4, 5) sont en métal, de préférence en acier, ou en un autre matériau ayant un bon couplage galvanique.

5. Compteur d'impulsions selon l'une quelconque des revendications précédentes, **caractérisé en ce que** les première et deuxième électrodes (4, 5) présentent une surface globalement plate pour un contact avec la peau du patient qui se trouve entre 7 et 19 cm².

6. Compteur d'impulsions selon l'une des revendications précédentes, **caractérisé en ce qu'**il comprend au moins un accéléromètre.

7. Compteur d'impulsions selon l'une des revendications précédentes, **caractérisé en ce qu'**il comprend un moyen pour imposer une fréquence entre les électrodes qui est sensiblement plus élevée que la fréquence utilisée pour la mesure d'impulsion.

8. Compteur d'impulsions selon l'une des revendications précédentes, **caractérisé en ce qu'**il comprend au moins un capteur de température.

9. Compteur d'impulsions selon l'une des revendications précédentes, **caractérisé en ce qu'**il comprend au moins un capteur de lumière pour détecter si le patient est couvert.

10. Compteur d'impulsions selon l'une des revendications précédentes, **caractérisé en ce qu'**il comprend un microphone, qui est adapté pour communiquer avec un récepteur à distance.

11. Compteur d'impulsions selon l'une des revendications précédentes, **caractérisé en ce qu'**il est connecté par un fil ou sans fil à une unité de base, laquelle unité de base comprend un moyen d'interface pour fournir un retour à un soignant de garde, tel qu'un moyen de génération d'affichage, de lumière ou de son.

12. Compteur d'impulsions selon la revendication 11, **caractérisé en ce qu'**il est adapté pour être fixé à l'unité de base quand il n'est pas utilisé, et **en ce qu'**il comprend un moyen pour activer ou désactiver l'alimentation en réponse au fait que le compteur d'impulsions est fixé à l'unité de base ou non, par exemple en mesurant l'impédance sur les première et deuxième électrodes.

13. Compteur d'impulsions selon l'une des revendications précédentes, **caractérisé en ce qu'**il comprend un moyen pour détecter une impédance sensiblement infinie entre les première et deuxième électrodes et pour désactiver le compteur d'impulsions si une impédance sensiblement infinie est détectée sur une certaine durée.

14. Compteur d'impulsions selon l'une des revendications précédentes, **caractérisé en ce qu'**il comprend un moyen pour désactiver le compteur d'impulsions si la bande de tension se trouve dans un mode non étiré pendant une durée prolongée.

15. Compteur d'impulsions selon l'une quelconque des revendications 4-16, **caractérisé en ce qu'**un actionneur (7) est connecté au côté intérieur de l'arc incurvé au niveau de deux sections de fixation, et dans lequel un interstice (11) est présent entre l'actionneur (7) et l'arc incurvé.

16. Compteur d'impulsions selon la revendication 15, **caractérisé en ce que** la troisième électrode (6) est agencée sur l'actionneur (7).

17. Compteur d'impulsions selon l'une quelconque des revendications précédentes, **caractérisé en ce que** ledit premier bras (1) et ledit deuxième bras (2) sont connectés à une articulation (12) au niveau de ladite partie centrale (3).

18. Compteur d'impulsions selon l'une quelconque des revendications 1 à 16, **caractérisé en ce que** ledit premier bras (1) et ledit deuxième bras (2) sont continus le long de ladite partie centrale (3).

19. Compteur d'impulsions selon l'une quelconque des revendications 1 à 17, **caractérisé en ce que** ledit premier bras (1) et ledit deuxième bras (2) présentent des extrémités supplémentaires respectives (14, 15) qui sont opposées aux premières extrémités (4a, 5a) et **en ce que** les premier et deuxième bras (1, 2) sont connectés l'un à l'autre avec une articulation (12) agencée entre les premières extrémités (4a, 5a) et les secondes extrémités (14, 15), dans lequel un ressort (16) est agencé entre le premier bras (1) et le deuxième bras (2) dans une position entre l'articulation (12) et les extrémités supplémentaires (14, 15).

20. Compteur d'impulsions selon l'une quelconque des revendications précédentes, **caractérisé en ce que** ledit premier bras (1) et ledit deuxième bras (2) sont disposés de façon symétrique autour de ladite partie centrale (3).

21. Compteur d'impulsions selon l'une quelconque des revendications précédentes, **caractérisé en ce que** les électrodes (4, 5, 6) sont électriquement connectées à un dispositif de sortie de communication sans fil.

22. Compteur d'impulsions selon l'une quelconque des revendications précédentes, **caractérisé en ce qu'**un dispositif d'affichage à sortie numérique (19) se trouve sur une face externe du compteur d'impulsions (10).
